# EUROPEAN PATENT APPLICATION

(11) **EP 4 290 149 A1**
(43) Date of publication of application: **13.12.2023**
(21) Application number: 22177572.9
(22) Date of filing: 07.06.2022
(51) Int. Cl.: F24F 11/61, F24F 11/70, A61M 21/00, H05B 45/20, F24F 110/10

(54) **METHOD AND DEVICE FOR IMPROVING COGNITIVE THERMAL COMFORT**

(71) Applicant: ABB SCHWEIZ AG, 5400 Baden (CH)
(72) Inventor: DOERSTEL, Bernhard Josef, 50321 Bruehl (DE); WARAKE, Ravindra, 411045 Balewadi, Pune (IN)
(74) Representative: Zimmermann & Partner Patentanwälte mbB

(57) **Abstract**

A method for improving the cognitive thermal comfort is presented in this invention. At least the color temperature of at least one light fixture is adjusted in accordance with a requested temperature setpoint, optionally also at least one HVAC device is adjusted in accordance with the requested temperature setpoint. Also, a method of upgrading at least one room to for improving the cognitive thermal comfort is presented in this invention. Furthermore, a light fixture color temperature adjustment device for improving the cognitive thermal comfort is presented in this invention.

## Description

### TECHNICAL AREA

The present disclosure relates to a method for improving the cognitive thermal comfort via adjusting the lighting color temperature within a room and an apparatus which consists of a heating-ventilation-air-conditioning (HVAC) system, and a lighting system, which is capable of adjusting the lighting color temperature, to improve the cognitive thermal comfort.

### TECHNICAL BACKGROUND

The perception of thermal comfort of a person derives from the primary and essential need to keep ones body temperature in a suitable and comfortable range. This body temperature range usually is within the bounds of 36°C to 38 °C. To ensure the thermal comfort of a person, it is necessary that the heat produced by a human body balances the heat released into the environment, and therefore, the thermal system of a human body gets to a steady state conditions from a thermal point of view. Thus, the thermal comfort sensation derives mainly from the balance or unbalance between the heat input and output of a human body.

In that respect, the human sensation of "cold" is a warning signal from and towards an individual that the heat lose is greater than the heat production of its human body. Subsequently, the human body is cooling down and experiences a net heat lose. Analogous, the human sensation of "hot" indicates, that the heat production of an individual human body is greater than the heat lose of it. Therefore, a net heat gain is present and the human body warms up.

While outdoor, the thermal conditions are dependent on climatic and weather conditions, indoor the temperature of rooms can be regulated by suitable means. Furthermore, to a certain extend the indoor temperatures are unaffected from the outdoor conditions and can be set and regulated within such bounds. Inside an air-conditioned building, the balance between heat production and heat lost by human body is achieved by supplying cooling or heating utilizing heating-ventilation-air-conditioning (HVAC) systems.

If a sensation of "cold" is present, a HVAC system can be triggered and controlled to either reduce the HVAC systems cooling energy supply or increase its heating energy supply. Analogous, the present of a "hot" sensation, and subsequent triggering and controlling of the HVAC system, lets the HVAC system either increase the cooling energy supply or reduce its heating energy supply.

Currently available systems are prone to be slow in achieving thermal comfort of the occupants of an indoor space, therefore, not fulfilling the needs of said occupants. The inventive idea of the present disclosure relates to a method and apparatus which are capable of greatly improving the cognitive thermal comfort of occupants in such surroundings.

### BRIEF DISCRIPTION

To improve existing HVAC systems a method and/or apparatus are needed which may be capable of improving the cognitive thermal comfort. This problem is solved by a method for improving the cognitive thermal comfort according to embodiments of the present disclosure. Also, the problem is solved by an apparatus according to embodiments of the present disclosure. Further embodiments, modifications and improvements will be apparent from the following description and the accompanying drawings and claims.

According to one aspect of the invention a method for improving the cognitive thermal comfort is provided, the method comprising:
- receiving a temperature setpoint via a temperature setpoint determining device;
- adjusting the color temperature of at least one light fixture within a first time frame according to the temperature setpoint.

According to another aspect of the invention a method for upgrading at least one room for improving the cognitive thermal comfort is provided, the method comprising:
- upgrading at least one lighting control device, the lighting control device being capable of receiving data from the illumination sensor and controlling the light fixture;
- optionally replacing at least one light fixture, the light fixture being capable of color temperature adjustments;
- optionally installing at least one illumination sensor, the illumination sensor being capable of detecting the illumination state and color temperature state within the at least one room;
- optionally upgrading at least one HVAC control device, the HVAC control device being capable of communicating with the lighting control device and controlling the HVAC device.

According to yet another aspect of the invention a light fixture color temperature adjustment device for improving the cognitive thermal comfort is provided, the device containing:
- at least one lighting control device, for controlling the color temperature of the at least one light fixture;
- wherein a temperature setpoint is received via a temperature setpoint determining device; and
- the color temperature of at least one light fixture is adjusted within a first time frame according to the temperature setpoint.

According to one advantageous aspect of the invention, the current and planned use state is determined for the at least one room within which the invention is applied.

According to one advantageous aspect of the invention, the adjustment of the room temperature of the at least one room via the HVAC device is limited while the color temperature of the at least one light fixture is adjusted according to the temperature setpoint, wherein limiting means that the limited temperature setpoint lays within the open temperature interval of the initial room temperature and the temperature setpoint.

According to one advantageous aspect of the invention, creating thermal perception within the occupying people via the color temperature adjustment of the at least one light fixture according to the temperature setpoint may decrease energy consumption and may increase occupant cognitive thermal comfort.

The invention will be further supported and described with reference to the following description and appended claims. The accompanying drawings, which are incorporated in and constitute a part of this specification, illustrate embodiments of the invention and, together with the description, serve to explain the principles of the invention.

### BRIEF DESCRIPTION OF THE FIGURES

A full and enabling disclosure of the present invention, including the best mode thereof, directed to one of ordinary skill in the art, is set forth in the specification, which makes reference to the appended figures.

In the following, the invention is explained in more detail with reference to embodiments, without these being intended to limit the scope of protection defined by the claims. The accompanying drawings illustrate embodiments and, together with the description, serve to explain the principles of the invention. The elements of the drawings are relative to each other and not necessarily to scale. Identical reference signs designate correspondingly similar parts.

The figures show:
Fig. 1: A flowchart of the method according to an embodiment of the present disclosure.
Fig. 2: A flowchart of the method according to an embodiment of the present disclosure.
Fig. 3: A schematic of a room which features a lighting system and a temperature setpoint device.
Fig. 4: A schematic of a room which features a lighting system, HVAC system and a temperature setpoint device.
Fig. 5: A schematic of the inter-connected lighting system and HVAC system via an inter-connect device.

### DETAILED DESCRIPTION

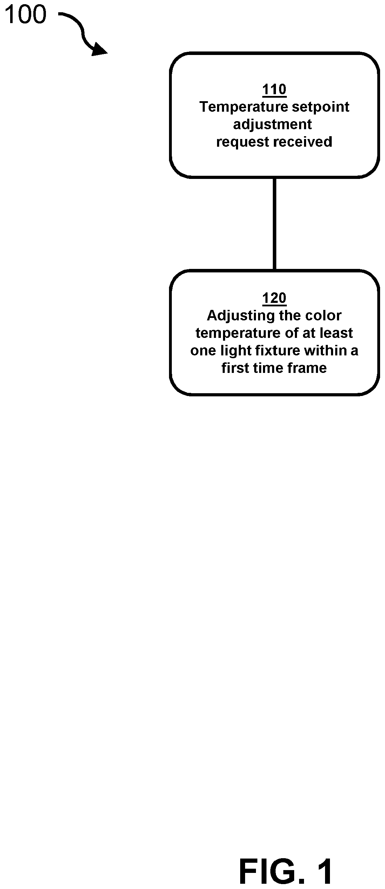

Figure 1 shows a flowchart of the method 100 according to an embodiment of the present disclosure. In the first method step a temperature setpoint adjustment request may be received 110. Herein, the temperature setpoint adjustment request may be initiated by any suitable means. In an embodiment, the temperature setpoint adjustment request may be determined by a temperature setpoint determining device 24. The temperature setpoint determining device 24 may be controlled by a user or by an automated system. In all cases, the control of the temperature setpoint device 24 may be manually triggered or via means of an intermediate control system. Also, in all cases, a hardware interface as well as a software interface may be used to interact with the user control and/or the automated system. The hardware interface may include, but is not limited to, switches, buttons, dials, keys, screens, touch screens, or levers. The software interface may include, but is not limited to control via a separate application (on the temperature setpoint device, with the automated system, or a sperate device like a smart phone).

In general, three different temperature setpoint adjustment requests are possible. Herein, a temperature setpoint adjustment request may be understood as either a request of a certain desired target temperature value or a relative temperature interval, relating to the currently present room temperature. Firstly, a request to lower the temperature of at least one rooms, e. g. cooling of the at least one room. Secondly, a request to increase the at least one rooms temperature, e. g. heating of the at least one room. And thirdly, a request to keep the at least one room temperature constant.

Still referring to Fig. 1, at method step 120 the lighting condition of the at least one light fixture 30 may be adjusted within a first time frame. The adjustment of the lighting condition of the at least one light fixture 30 may include, but is not limited to, the adjustment of color temperature, color, or brightness of the at least one light fixture 30. In an embodiment at least the color temperature of the at least one light fixture 30 may be adjusted.

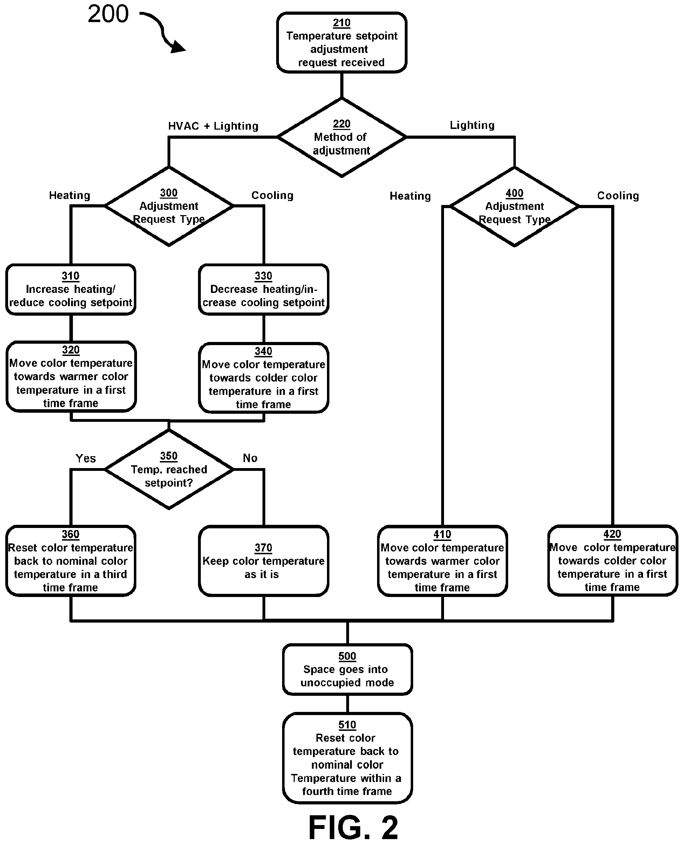

Figure 2 shows a flowchart of the method 200 according to an embodiment of the present disclosure. In the first method step a temperature setpoint adjustment request may be received 210. Herein, the temperature setpoint adjustment request may be initiated by any suitable means. In an embodiment, the temperature setpoint adjustment request may be determined by a temperature setpoint determining device 24. The temperature setpoint determining device 24 may be controlled by a user or by an automated system. In all cases, the control of the temperature setpoint device 24 may be manually triggered or via means of an intermediate control system. Also, in all cases, a hardware interface as well as a software interface may be possible to interact with the user control and/or the automated system. The hardware interface may include, but is not limited to, switches, buttons, dials, keys, screens, touch screens, or levers. The software interface may include, but is not limited to, control via a separate application (on the temperature setpoint device, within the automated system, or a separate device like a smart phone).

Based on the current and planned use state of the at least one room, within which the method 200 may be applied, a method of adjustment may be determined 220. The current and planned use state of the at least one room may be determined by any suitable means. In an embodiment, the planned use state of the at least one room may be determined by the rooms type (e. g. meeting room, office, storage room, hallway, toilet) and/or by a room scheduling method.

In the case of a room type, which has in general a short occupancy duration of 15 minutes or less, like a storage room, for this room a "short occupancy flag" may be determined and saved. In the case of a room type, which has in general a long occupancy duration of 15 minutes or more, like an office, for this room a "long occupancy flag" may be determined and saved. In case of a room type, which can have a short or long occupancy duration, like a meeting room, for this room a "intermediate occupancy flag" may be determined and saved.

The occupancy duration, which is used to determine an "occupancy flag" is adjustable. Therefore, the above-mentioned 15 minutes or less for a "short occupancy flag" or the 15 minutes and more for a "long occupancy flag" are controllable and adjustable within the method and apparatus laid out herein.

For all rooms a room scheduling method may be applied. This room scheduling method may be limited to the said room and/or inter-connected with a room scheduling method for other rooms and/or the whole building. Also, the room scheduling method may be controlled by a room scheduling device of the at least one room and/or by an inter-connected method which determines the room scheduling form other sources, like planned occupation of the at least one room by room occupants. In embodiments, the room scheduling method determines the planned occupation duration of the at least one room based on the input of the room scheduling device. For instance, either a short occupation duration or a long occupation duration may be determined and subsequently labeled with a "short occupation flag" or a "long occupation flag" respectively.

Based on the "occupation flag", either "short occupation flag" or "long occupation flag", the method of adjustment 220 may choose between at least two thermal comfort adjustment options. In an embodiment, a choice of two thermal comfort adjustment options may be present.

If a "short occupation flag" is determined, the current state of the HVAC system may not be changed. In an embodiment, the HVAC system may control the HVAC device 20 in such a way, that the temperature setpoint which was present before an adjustment request via the temperature setpoint determining device 24 was received, may be maintained. Also, in presents of a "short occupation flag", the lighting conditions of a at least one light fixture 30 may be adjusted.

If a "long occupation flag" is determined, the current state of the HVAC system may be adjusted. Also, in the presents of a "long occupation flag", the lighting conditions of the at least one light fixture 30 may be adjusted.

Still relating to Fig. 2, and if a "long occupation flag" is present, the method of adjustment 220 may choose a simultaneous adjustment of the current condition of the at least one HVAC device 20 and the at least one light fixture 30.

Through a temperature sensor 22 the room temperature may be constantly determined. Furthermore, the method 200 may constantly compares the requested temperature setpoint with the current room temperature.

In the presents of a "long occupation flag", and a simultaneous adjustment of the current condition of the at least one HVAC device 20 and the at least one light fixture 30 in method step 300 it may be determined which type of temperature setpoint adjustment may be requested.

If a heating of the at least one room is requested at method step 310, the HVAC device 20 may be controlled in a way that its current cooling condition may be reduced or its current heating condition may be increased within a second time frame. Furthermore, at method step 320, the lighting condition of the at least one light fixture 30 may be adjusted. The adjustment of the lighting condition of the at least one light fixture 30 may include, but is not limited to, the adjustment of color temperature, color, or brightness of the at least one light fixture 30. In an embodiment, at least the color temperature of the at least one light fixture 30 may be adjusted. Since the temperature setpoint adjustment request indicates that a heating of the at least one room is requested, the color temperature of the at least one light fixture 30 may be adjusted within a first time frame towards a warmer color temperature.

If a cooling of the at least one room is requested at method step 330 the HVAC device 20 may be controlled in a way, that its current cooling condition may be increased or its current heating condition may be reduced within a second time frame. Furthermore, at method step 340, the lighting condition of the at least one light fixture 30 may be adjusted. The adjustment of the lighting condition of the at least one light fixture 30 may include, but is not limited to, the adjustment of color temperature, color, or brightness of the at least one light fixture 30. In an embodiment, at least the color temperature of the at least one light fixture 30 may be adjusted. Since the temperature setpoint adjustment request indicates that a cooling of the at least one room is requested, the color temperature of the at least one light fixture 30 may be adjusted within a first time frame towards a colder color temperature.

Through the constant comparison of the requested temperature setpoint and the current room temperature, for instance determined by the temperature sensor 22, the method 100 may be capable of determining if and when the requested temperature setpoint is reached.

If the requested temperature setpoint is yet not reached 350, the initiated adjustment of the at least one HVAC device 20 condition and the adjustment of the at least one light fixture 30 may be kept at their adjusted state 370.

If the requested temperature setpoint is reached 350, the initiated adjustment of the at least one HVAC device 20 may be kept at its adjusted state, while the adjustment of the at least one light fixture 30 may be reset within a third time frame to a nominal condition of the light fixture 30 360. Herein, nominal condition of the light fixture 30 can be any predefined lighting condition of the at least one light fixture 30. In an embodiment, in the nominal condition of the at least one light fixture 30 the lighting conditions correspond to the lighting conditions before the adjustment took place. This may include, but is not limited to, the at least one light fixture 30 color temperature, color, or brightness.

Still relating to Fig. 2, and if a "short occupation flag" is present, the method of adjustment 220 may choose only an adjustment of the current condition of the at least one light fixture 30.

In the presents of a "short occupation flag", in method step 400 it may be determined which type of temperature setpoint adjustment is requested and a subsequent adjustment of the at least one light fixture 30 may be performed.

If a heating of the at least one room is requested at method step 410 the lighting condition of the at least one light fixture 30 may be adjusted. The adjustment of the lighting condition of the at least one light fixture 30 may include, but is not limited to, the adjustment of color temperature, color, or brightness of the at least one light fixture 30. In an embodiment, at least the color temperature of the at least one light fixture 30 may be adjusted. Since the temperature setpoint adjustment request indicates that a heating of the at least one room is requested, the color temperature of the at least one light fixture 30 may be adjusted within a first time frame towards a warmer color temperature.

If a cooling of the at least one room is requested at method step 420 the lighting condition of the at least one light fixture 30 may be adjusted. The adjustment of the lighting condition of the at least one light fixture 30 may include, but is not limited to, the adjustment of color temperature, color, or brightness of the at least one light fixture 30. In an embodiment, at least the color temperature of the at least one light fixture 30 may be adjusted. Since the temperature setpoint adjustment request indicates that a cooling of the at least one room is requested, the color temperature of the at least one light fixture 30 may be adjusted within a first time frame towards a colder color temperature.

In the event of the at least one room experiencing an unoccupied state 500, the temperature setpoint may be adjusted to a predefined temperature setpoint, and subsequently a corresponding control of the at least one HVAC device 20 may be set. Furthermore, the lighting control may reset the at least one light fixture 30 within a fourth time frame to a nominal condition 510. In an embodiment, the reset to the nominal condition of the at least one light fixture 30 may be performed within a fourth time frame which tends to an instantaneous reset.

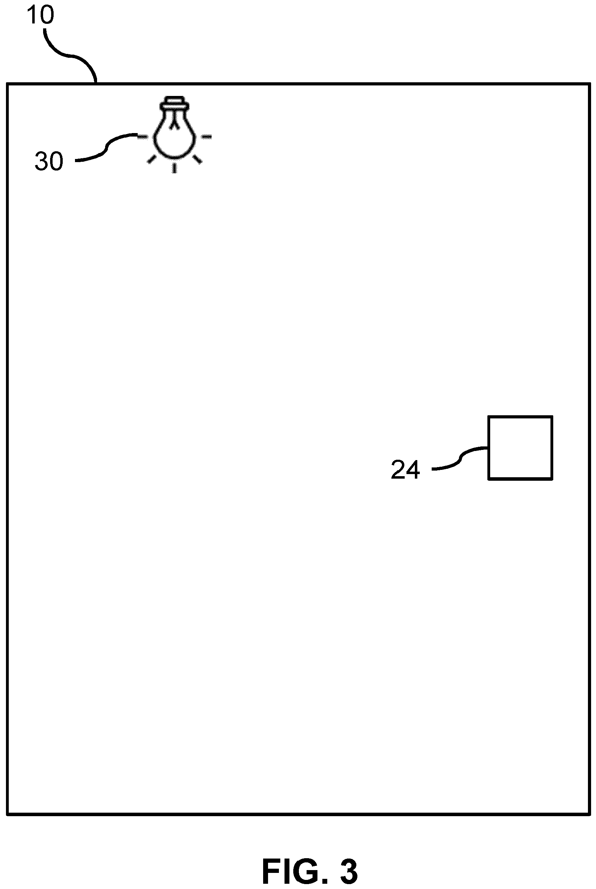

Figure 3 shows a schematic of at one room 10 according to embodiments described herein. The at least one room 10 may contain at least one lighting system and at least one temperature setpoint device 24.

The at least one lighting system may contain at least one light fixture 30 which may be inter-connected via at least one lighting control device 44, 46 (not shown in Fig. 3).

The at least one light fixture 30 may be any suitable illumination device, like incandescent light bulbs, light-emitting diodes (LED), arc lamps, or gas-discharge lamps. Furthermore, the at least one light fixture 30 may be placed within the at least one room 10 in such a way that it is capable of illuminating at least a part of the at least one room 10. In an embodiment, the at least one light fixture 30 may be placed within the at least one room 10 in such a way, that it is capable of illuminating large portions of the at least one room 10, in its extreme, all of the at least one room 10. Also, the at least one light fixture 30 may be capable of adjustment option with regards to its illumination, like color temperature, color, or brightness of the emitted light of the at least one light fixture 30. The at least one light fixture 30 may support a suitable range of adjustment of all available illumination parameters. Hereby, in an embodiment the color temperate may range from 2000 K to 6500 K, the color may range from white to yellow, and the brightness may range from 500 Lumen to 10000 Lumen. Each of the at least one light fixtures 30 may be controlled individually.

Furthermore, the at least one room 10 may contain a temperature setpoint device 24. The temperature setpoint determining device 24 may be controlled by a user or by an automated system. In all cases, the control of the temperature setpoint device 24 may be manually triggered or via means of an intermediate control system. Also, in all cases, a hardware interface as well as a software interface may be possible to interact with the user control and/or the automated system. The hardware interface may include, but is not limited to, switches, buttons, dials, keys, screens, touch screens, or levers. The software interface may include, but is not limited to, control via a separate application (on the temperature setpoint device, within the automated system, or a separate device like a smart phone).

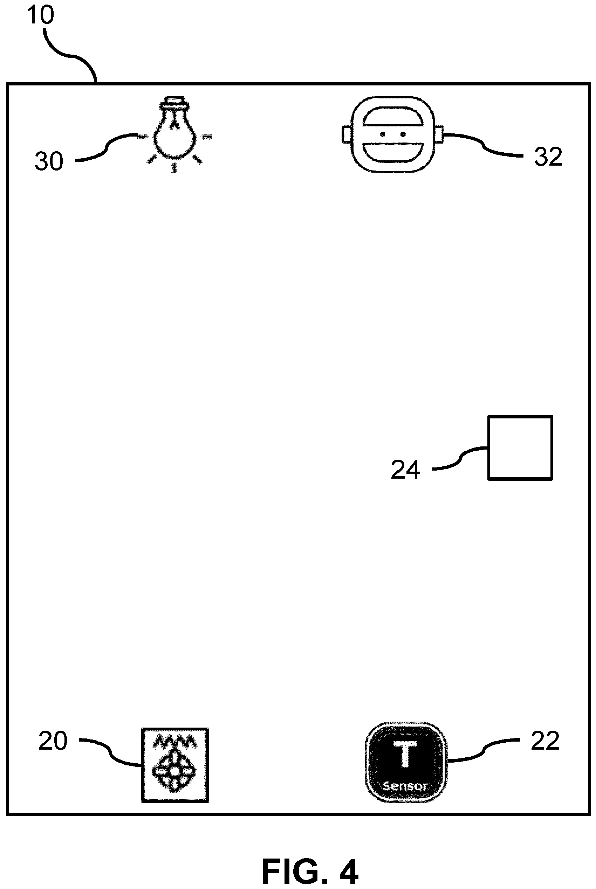

Figure 4 shows a schematic of at least one room 10 according to embodiments described herein. The at least one room 10 may contain at least one lighting system, at least one HVAC system, and at least one temperature setpoint device 24.

The at least on lighting system contains at least one light fixture 30 and at least one illumination sensor 32 which may be inter-connected via a at least one lighting control device 44, 46 (not shown in Fig. 4). The at least one light fixture 30 may be any suitable illumination device, like incandescent light bulbs, light-emitting diodes (LED), arc lamps, or gas-discharge lamps. Furthermore, the at least one light fixture 30 may be placed within the at least one room 10 in such a way that it may be capable of illuminating at least a part of the at least one room 10. In an embodiment, the at least one light fixture 30 may be placed within the at least one room 10 in such a way, that it may be capable of illuminating large portions of the at least one room 10, in its extreme, all of the at least one room 10. Also, the at least one light fixture 30 may be capable of adjustment option with regards to its illumination, like color temperature, color, or brightness of the emitted light of the at least one light fixture 30. The at least one light fixture 30 may support a suitable range of adjustment of all available illumination parameters. Hereby, in an embodiment the color temperate may range from 2000 K to 6500 K, color may range from white to yellow and the brightness may range from 500 Lumen to 10000 Lumen. Each of the at least one light fixtures 30 may be controlled individually.

The at least one illumination sensor 32 within the at least one room 10 may be capable of determining multiple illumination parameters of the illumination within the at least one room 10. These detectable illumination parameters may be, but not limited to, color temperature, color, and/or brightness of the emitted light within the at least one room 10. The at least one illumination sensor 32 may be placed within the at least one room 10 in such a way, that the at least one illumination sensor 32 may be capable of detecting at least a fraction of the total emitted light within the at least one room 10. In an embodiment, the at least one illumination sensor 32 may be placed within the at least one room 10 in such a way, that the least one illumination sensor 32 may be capable of detecting a representative fraction of the total emitted light within the at least one room 10.

The at least one HVAC system may contain at least one HVAC device 20 and at least one temperature sensor 22 which may be inter-connected via a at least one HVAC control device 42 (not shown in Fig. 4). The at least one HVAC device 20 may be a self-sufficient HVAC device, or a part of a larger scale floor or building HVAC device. In the case the HVAC device is part of a larger scale floor or building HVAC device, the HVAC device 20 may only consist of ducting, at least one inlet and/or at least one outlet within the at least one room 10 which are connected to the larger scale floor or building HVAC device. The HVAC device 22 may be capable of heating, ventilating, and air-conditioning (cooling) the at least one room 10. The HVAC device 20 thereby may support a suitable temperature range of operation which may lay within the range of - 50 °C to +70 °C. Also, the HVAC device 20 may support a suitable range of ventilation which may range from 5 CFM to 10 CFM of air.

The at least one temperature sensor 22 within the at least one room 10 may be capable of determining the room temperature of the at least one room 10.

Furthermore, the at least one room 10 may contain a temperature setpoint device 24. The temperature setpoint determining device 24 may be controlled by a user or by an automated system. In all cases, the control of the temperature setpoint device 24 may be manually triggered or via means of an intermediate control system. Also, in all cases, a hardware interface as well as a software interface may be possible to interact with the user control and/or the automated system. The hardware interface may include, but is not limited to, switches, buttons, dials, keys, screens, touch screens, or levers. The software interface may include, but is not limited to, control via a separate application (on the temperature setpoint device, within the automated system, or a separate device like a smart phone).

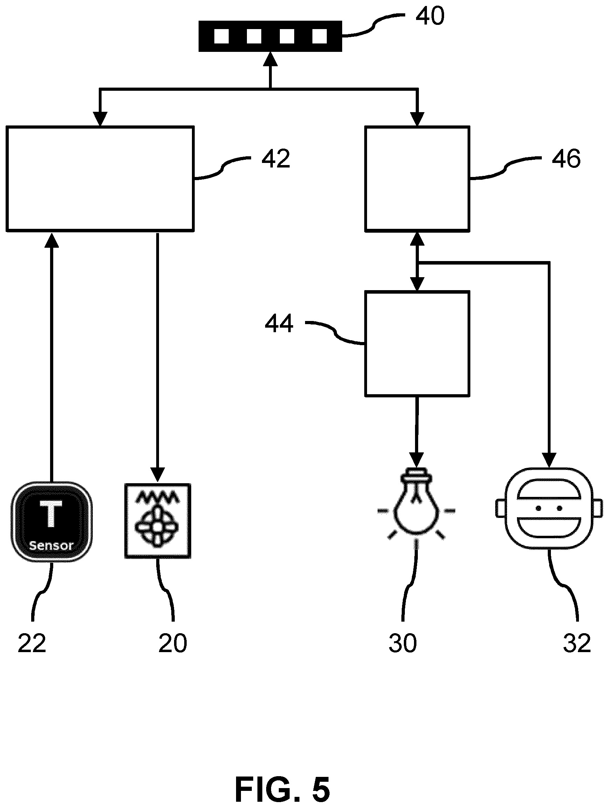

Figure 5 shows a schematic of the inter-connected lighting system and HVAC system via an inter-connect device 40. The at least one lighting system may contain at least one light fixture 30, at least one illumination sensor 32, and at least one lighting control device 44, 46. The at least one HVAC system may contain at least one HVAC device 20, at least one temperature sensor 22, and at least one HVAC control device 42.

The light fixture 30 may be any suitable light fixture which is capable of illuminating its surrounding, like illuminants, lamps, arc lamps, fluorescent lamps, fuel lamps, gas-discharge lamps, incandescent lamps, plasma lamps, light-emitting diodes (LED).

Within the lighting system the light fixture 30 may be inter-connected to at least a first lighting control device 44 and 46 or 46 only. Also, within the lighting system the illumination sensor may be inter-connected to at least a second lighting control device 44 and 46 or 46 only. Furthermore, all of the lighting control devices may be inter-connected to one another. At least one of the lighting control devices 44, 46 may be interconnected via the inter-connect device 40.

The first lighting control device 44 or 46 may be capable of controlling the at least one light fixture 30. Herein, the first lighting control device 44 or 46 may control all illumination parameters of the light fixture 30 which may be, but are not limited to, color temperature, color, or brightness of the light fixture 30.

The second lighting control device 44 and 46 or 46 only may be capable of receiving illumination parameters from the at least one illumination sensor 32. Herein, the second lighting control device 44 and 46 or 46 only may receive, but is not limited to, color temperature, color, or brightness determined by the at least one illumination sensor 32.

Via the inter-connection of the first lighting control device 44 and 46 or 46 only and second lighting control device 44 and 46 or 46 only the at least one light fixture 30 may be controlled based on the illumination parameters determined by the illumination sensor 32.

Still referring to Fig. 5, the HVAC system is shown. Within the HVAC system the at least one HVAC device 20 and the at least one temperature sensor 22 may each be inter-connected to the HVAC control device 42. The HVAC control device 42 may be capable of receiving temperature parameters from the at least one temperature sensor 22. Also, the HVAC control device 42 may be capable of controlling the at least one HVAC device 20, which includes, but is not limited to, heating, ventilating, and air-conditioning (cooling).

Via the HVAC control device 42 the at least one HVAC device 20 may be controlled based on the received temperature parameters from the at least one temperature sensor 22.

Via the inter-connect device 40 the lighting system and HVAC system may be inter-connected. Therefore, the HVAC control device 42 may be capable of receiving illumination parameters from the lightning system and at least one lighting control device 44, 46 may be capable of receiving temperature parameters from the HVAC system.

In an embodiment, the HVAC control device 42 may be capable of controlling the lighting system based on the received temperature parameters and the received illumination parameters.

In another embodiment, at least one lighting control device 44, 46 may be capable of controlling the HVAC system based on the received lighting parameters and the received illumination parameters.

In still another embodiment, the lighting system and the HVAC system may be both controlled by a separate control system (not shown in Fig. 5) which may be situated on-site or off-site of the at least one room 10 containing building. In all cases, the illumination parameters and the temperature parameters may be transmitted to the separate control system via the at least one lighting control device 44, 46, the HVAC control device 42 and the inter-connect device 40.

### DISCUSSION OF FUTHER DETAILS, POSSIBLE VARIANTS AND COMMON ASPECTS

Possible variants and general optional aspects of the invention are further described. Here, unless excluded, any aspect may be combined with any other aspect of the invention. The aspects are also referred to as embodiments and are illustrated in part by reference signs which refer to the elements shown in the figures previously described, but without being limited in any further respect to the embodiments shown therein.

In a selected embodiment, a method 100 for improving the cognitive thermal comfort is provided, the method comprising receiving a temperature setpoint via a temperature setpoint determining device 24, adjusting the color temperature of at least one light fixture 30 within a time frame according to the temperature setpoint 320, 340, 410, 420.

Generally, a temperature of the surrounding of about 18 °C to 26 °C is wanted for a human being to feel well and comfortable, and therefore are perceived as "normal" or "neutral". Thus, temperatures of the surrounding which are less than 18 °C are perceived as "cool" or "cold", while temperatures of the surrounding which exceed 26°C are perceived as "warm" or "hot".

The cognitive thermal comfort can be influenced by numerous factors. Two of these factors are the temperature of the surrounding and the color temperature of the dominant light source. Herein, dominant light source is understood as the light perceived by a human being, which can be emitted by one or more light sources.

The perception of cognitive thermal comfort can correspond to the actual temperature of the surrounding, but not has to. It is possible to create a perception of cognitive thermal comfort through the means of controlling the color temperature of the dominant light source even if the temperature of the surrounding is actually "to warm" or "hot" or "to cool" or "cold" and therefore, having a perception of cognitive thermal comfort which does not correspond to the actual temperature of the surrounding.

Generally, a color temperature of dominant light source of about 3000 K or lower is perceived "warm", a color temperature of the dominant light source of about 4000 K is perceived as "neutral" or "normal", and a color temperature of the dominant light source of about 5000 K or higher is perceived "cool".

In the case the temperature of the surrounding is to cold, and subsequently a human being is feeling cold, by changing the color temperature of the dominant light source towards a warmer color temperature, therefore towards 2000 K, a perception of "warm" within the human being can be achieved, even if the actual temperature of the surrounding does not support this perception.

Analogous, in the case the temperature of the surrounding is to warm, and subsequently a human being is feeling warm, by changing the color temperature of the dominant light source towards a colder color temperature, therefore towards 6500 K, a perception of "cool" within the human being can be achieved, even if the actual temperature of the surrounding does not support this perception.

For surroundings, which are capable of controlling the temperature of the surrounding as well as at least the color temperature of the dominant light source, this phenomenon of perceived cognitive thermal comfort may be utilized.

When a temperature setpoint adjustment for the surrounding is received via a temperature setpoint determining device 24, which indicates, that the actual temperature of the surrounding at the moment is not within the bounds of thermal comfort, the color temperature of at least one light fixture 30 may be adjusted within a first time frame in accordance with the received temperature setpoint 120, 320, 340, 410, 420.

The first time frame for adjusting the color temperature may be chosen in such a way, that a human being occupying the surrounding is not irritated by the color temperature adjustment. An irritation may be present, when the first time frame for adjusting the color temperature is chosen which is too short. In an embodiment possible time frames for adjusting the color temperature may range from 10 seconds or more to 600 seconds, and are preferably greater or equal to 60 seconds. It has to be noted, that the given time frames can be controlled and adjusted within the method and apparatus laid out herein.

In an embodiment, the current and planned use state of at least one room 10 within which the method 100 200 for improving the cognitive thermal comfort is applied is determined.

In an embodiment, a thermal comfort adjustment option may be chosen, based on the current and planned use state of the at least one room 10 210.

Based on the "occupation flag", either "short occupation flag" or "long occupation flag", one of at least two thermal comfort adjustment options may be chosen. In an embodiment, a choice of two thermal comfort adjustment options may be present.

In an embodiment, the current state of the ate least one HVAC device may not be changed.

In an embodiment, the temperature of the at least one room 10 may be adjusted via at least one HVAC device 20 within a second time frame according to the temperature setpoint 310, 330.

If the requested temperature setpoint lays above the actual and currently present room temperature, e. g. heating request, the at least one HVAC device 20 may be controlled in a way, that its current cooling condition may be reduced or its current heating condition may be increased. If the requested temperature setpoint lays below the actual and currently present room temperature, e. g. cooling request, the at least one HVAC device 20 may be controlled in a way, that its current cooling condition may be increased or its current heating condition may be reduced.

The adjustment of the at least one HVAC device 20 may be carried out within a second time frame. In an embodiment the second time frame may be chosen such, that an occupant of the at least one room 10 may not be irritated by the adjustment of the at least one HVAC device 20, and the subsequent change of the room temperature.

In an embodiment, the color temperature of the at least one light fixture 30 may be adjusted to its nominal color temperature within a third time frame, when adjustment of the temperature of the at least one room 10 via a HVAC device 20 has reached the temperature setpoint within a predefined margin 360.

Through a temperature sensor 22 the room temperature may be constantly determined. Furthermore, the requested temperature setpoint may be constantly compared with the current room temperature. When the actual and currently present room temperature and the requested temperature setpoint lay within a predefined margin, the color temperature of the at least one light fixture 30 may be reset to a nominal color temperature within a third time frame.

Herein, a nominal color temperature is understood as a predefined color temperature. The predefined nominal color temperature may lay within a margin to the normal or natural color temperature of about 4000 K, like from 3000 K to 5000 K. In an embodiment, the predefined nominal color temperature may lay within a margin to the normal or natural color temperature of about 4000 K, like from 3500 K to 4500 K.

The reset of the color temperature of the at least one light fixture 30 to its nominal color temperature may be performed within a third time frame. The third time frame for adjusting the color temperature may be chosen in such a way, that a human being occupying the at least one room 10 may not be irritated by the color temperature adjustment. An irritation can be present, when the time frame for adjusting the color temperature is chosen which is too short. In an embodiment possible third time frames for adjusting the color temperature may range from 10 seconds or more to 600 seconds, and may be preferably greater or equal to 60 seconds. It has to be noted, that the given time frames can be controlled and adjusted within the method and apparatus laid out herein.

In an embodiment, the color temperature of the at least one light fixture 30 may be adjusted to its nominal color temperature with in a fourth time frame, when it is determined that the current use state of the at least one room 10 changes to an unused use state 510.

The reset of the color temperature of the at least one light fixture 30 to its nominal color temperature may be performed within a fourth time frame. Since the current use state indicates an unused use state, the reset of the color temperature of the at least one light fixture 30 may be chosen without considering possible irritation of human beings. In an embodiment possible fourth time frames for adjusting the color temperature may range from 10 seconds or more to 600 seconds, and may be preferably greater or equal to 60 seconds. It has to be noted, that the given time frames can be controlled and adjusted within the method and apparatus laid out herein.

When a "short occupation flag" is determined, and an additional (second) adjustment request via the temperature setpoint determining device 24 is received within a fifth time frame after the an initial (first) adjustment request via the temperature setpoint determining device 24, the HVAC system may control the HVAC device 20 in such a way, that the HVAC device 20 will provide heating or cooling energy according the additional (second) adjustment request. The fifth time frame may range from 1 minute or more to 20 minutes or less after the initial (first) adjustment request was received, and may preferably range from 5 minutes or more to 15 minutes or less. It has to be noted, that the given time frames can be controlled and adjusted within the method and apparatus laid out herein.

In an embodiment, a method for upgrading at least one room 10 for improving the cognitive thermal comfort is presented, the method comprising installing or replacing at least one light fixture 30, the light fixture 30 being capable of color temperature adjustments, installing or replacing at least one illumination sensor 32, the illumination sensor 32 may be capable of detecting the illumination state and color temperature state within the at least one room 10, installing or replacing at least one lighting control device 44, 46, the lighting control device 44, 46 may be capable of receiving data from the illumination sensor 32 and controlling the light fixture 30, installing or replacing at least one HVAC control device 42, the HVAC control device 42 may be capable of communicating with the at least one lighting control device 44, 46 and controlling the HVAC device 20.

If an existing room 10 does not feature all the necessary hardware and software equipment to apply a method for improving the cognitive thermal comfort, the relevant hardware and software components may be replaced with hardware and software components which may be capable of applying a method for improving the cognitive thermal comfort.

If no light fixture 30 is present, at least one light fixture 30 may be installed. If at least one light fixture 30 is present, but is not capable of being controlled in terms of, but not limited to, color temperature, color, or brightness of the least one light fixture 30 may be replaced with a light fixture 30 which is capable of being controlled in terms of, but not limited to, color temperature, color, or brightness.

If no illumination sensor 32 is present, at least one illumination sensor 32 may be installed. If at least one illumination sensor 32 is present, but not capable of determining illumination parameters like color temperature, color, or brightness the at least one illumination sensor 32 may be replaced with at least one illumination sensor 32 which is capable of determining illumination parameters like color temperature, color, or brightness.

If no lighting control device 44, 46 is present, at least one lighting control device 44, 46 may be installed. If at least one lighting control device 44, 46 is present but not capable of receiving data from the at least one illumination sensor 32 and controlling the at least one light fixture 30, the at least one lighting control device 44, 46 may be replaced with at least one lighting control device 44, 46 which is capable of receiving data form the at least one illumination sensor 32 and controlling the at least one light fixture 30.

If no HVAC control device 42 is present, at least one HVAC control device 42 may be installed. If at least one HVAC control device 42 is present but not capable of communicating with the at least one lighting control device 44, 46 and controlling the HVAC device 20, the HVAC control device 42 may be replaced with at least one HVAC control device 42 which is capable of communicating with the at least one lighting control device 44, 46 and controlling the HVAC device 20.

If no HVAC device 20 is present, at least one HVAC device 20 may be installed. If not temperature sensor 22 is present, at least one temperature sensor 22 may be installed.

In an embodiment, the color temperature of the at least one light fixture 30 may be adjusted within a first time frame according to the temperature setpoint, the 10 seconds or more to 600 seconds, and may be preferably greater or equal to 60 seconds. It has to be noted, that the given time frames can be controlled and adjusted within the method and apparatus laid out herein. Within a first time frame does in particular not include an immediate adjustment of the color temperature.

In an embodiment, the temperature of the at least one room 10 may be adjusted via the HVAC device 20 within a second time frame according to the temperature setpoint, the second time frame may be between 10 seconds or more to 1800 seconds. It has to be noted, that the given time frames can be controlled and adjusted within the method and apparatus laid out herein.

In an embodiment, the adjustment of the room temperature of the at least one room 10 via the HVAC device 20 may be limited to a limited temperature setpoint, the limited temperature setpoint being within an open temperature interval of the initial room temperature and the requested temperature setpoint, while the color temperature of the at least one light fixture 30 may be adjusted according to the requested temperature setpoint.

When a temperature setpoint request is received via the temperature setpoint determining device 24, the adjustment of the room temperature of the at least one room 10 via the HVAC device 20 may be limited. Herein, limited is understood that the targeted room temperature lays within an open temperature interval of the initial room temperature and the requested temperature setpoint. Therefore, in case a heating of the at least one room 10 is requested, the targeted room temperature, which may also be used to control the at least one HVAC device 20, may be lower than the requested temperature setpoint. In case a cooling of the at least one room 10 is requested, the targeted room temperature, which may also be used to control the at least one HVAC device 20, may be higher than the requested temperature setpoint.

Therefore, the energy and power consumption of the HVAC device 20 may be reduced, which in turn proves advantageous in terms of cost of operations, cost of acquisition, and environmental impact of the at least one HVAC device 20.

In case the temperature adjustment via the HVAC device 20 may be limited, the requested temperature setpoint may not be reachable. Therefore, a simultaneous adjustment of the color temperature of the at least one light fixture 30 in accordance with the requested temperature setpoint may be performed. If a heating of the at least one room 10 is requested, the color temperature of the at least one light fixture 30 may be adjusted towards warmer color temperatures. If a cooling of the at least one room 10 is requested, the color temperature of the at least one light fixture 30 may be adjusted towards colder color temperatures.

Therefore, even when the requested temperature setpoint is not reached or not reachable, the perception and cognitive thermal comfort of the occupants of the at least one room 10 may be influenced towards the requested temperature setpoint.

This method may also be applied, if in the event of HVAC device 20 failure, the temperature adjustment via the HVAC device 20 may not intentionally limited.

In an embodiment, the method for improving the cognitive thermal comfort may be controlled on premises of the at least one room 10 or at a remote location like on a cloud platform.

Therefore, the data from the lighting system and HVAC system may be collected and processed by the respective at least one lighting control device 44, 46 and HVAC control device 42 or from at least one off-site control device. Also, the control of the lighting system and HVAC system may be controlled by the respective at least one lighting control device 44, 46 and HVAC control device 42 of from at least one off-site control device.

In an embodiment, the control of the at least one HVAC device 20 may be performed by the at least one lighting control device 44, 46, wherein the at least one lighting control device 44, 46 may receive all data and parameters from the at least one HVAC device 20, the at least one temperature sensor 22, the at least one temperature setpoint device 24, the at least one light fixture 30, and the at least one illumination sensor 32.

In an embodiment, the control of the at least one light fixture 30 may be performed by the at least one HVAC control device 42, wherein the at least HVAC control device 42 may receive all data and parameters from the at least one HVAC device 20, the at least one temperature sensor 22, the at least one temperature setpoint device 24, the at least one light fixture 30, and the at least one illumination sensor 32.

In an embodiment, the control of the at least one light fixture 30 and the at least one HVAC device 20 may be performed by at least one multi-purpose control device, wherein the at least one multi-purpose control device may receive all data and parameters from the at least one HVAC device 20, the at least one temperature sensor 22, the at least one temperature setpoint device 24, the at least one light fixture 30, and the at least one illumination sensor 32.

If at least one off-site control device is utilized for data collection, processing and controlling of the lighting system and HVAC system, the at least one off-site control device is capable of receiving and processing all above mentioned date from the lighting system and the HVAC system. Furthermore, the at least one off-site control device may be capable of controlling the lighting system and HVAC system in way laid out above.

In a selected embodiment, a light fixture color temperature adjustment device for improving the cognitive thermal comfort is presented, the device may contain at least one lighting control device 44, 46, wherein the color temperature of the at least one light fixture 30 is controlled by the at least one lighting control device 44, 46, wherein a temperature setpoint is received via a temperature setpoint determining device 24, and the color temperature of at least one light fixture 30 is adjusted within a time frame according to the temperature setpoint.

In an embodiment, the light fixture color temperature adjustment device may also comprise at least one or more temperature setpoint determining device 24 and/or light fixture 30.

In an embodiment, the light fixture color temperature adjustment device may also comprise at least one or more illumination sensor 32, temperature sensor 22, HVAC device 20, interconnect device 40, HVAC control device 42, wherein the room temperature of the at least one room 10 may be adjusted by the at least one HVAC device 20 which may be controlled by the at least one HVAC control device 42, wherein the interconnect device 40 may interconnect the HVAC control device 42 and the lighting control device 44, 46.

In an embodiment, the current and planned use state of at least one room 10 within which the device is applied may be determined.

### REFERENCE NUMERALS

- 10: Room
- 20: HVAC device
- 22: Temperature sensor
- 24: Temperature setpoint device
- 30: Light fixture
- 32: Illumination sensor
- 40: Inter-connect device
- 42: HVAC control device
- 44: (First) Lighting control device
- 46: (Second) Lighting control device

## Claims

1. A method (100, 200) for improving the cognitive thermal comfort in at least one room (10), the method comprising:
receiving (110, 210) a temperature setpoint via a temperature setpoint determining device (24); and
adjusting the color temperature of at least one light fixture (30) within a first time frame according to the temperature setpoint (120, 320, 340, 410, 420).

2. A method of claim 1, the method further comprising, determining the current and/or planned use state of the at least one room (10) within which the method is applied.

3. A method of claim 2, the method further comprising, choosing a thermal comfort adjustment option based on the current and/or planned use state of the at least one room (220).

4. A method of any preceding claims, the method further comprising, adjusting the temperature of the at least one room (10) via at least one heating-ventilation-air-conditioning (HVAC) device (20) within a second time frame according to the temperature setpoint (310, 320).

5. A method to any preceding claims, the method further comprising, the first time frame being less or equal to the second time frame.

6. A method of claim 4, the method further comprising, adjusting the color temperature of the at least one light fixture (30) to its nominal color temperature within a third time frame, when adjustment of the temperature of the at least one room (10) via a HVAC device (20) has reached the temperature setpoint within a predefined margin (360).

7. A method of any preceding claims, the method further comprising, adjusting the color temperature of the at least one light fixture (30) to its nominal color temperature with in a fourth time frame, when determining the current use state of the at least one room (10) determines an unused use state (510).

8. A method of any preceding claims, the method further comprising, adjusting the color temperature of the at least one light fixture (30) within a first time frame according to the temperature setpoint, the first time frame being greater than 10 seconds and/or lower than 600 seconds, while the limits of the first time frame are user adjustable.

9. A method of any preceding claims, the method further comprising, adjusting the temperature of the at least one room (10) via the HVAC device (20) within a second time frame according to the temperature setpoint, the second time frame being greater than 1 minute and/or lower than 30 minutes, while the limits of the second time frame are user adjustable.

10. A method of any preceding claims, the method further comprising, limiting the adjustment of the room temperature of the at least one room (10) via the HVAC device (20) to a limited temperature setpoint, the limited temperature setpoint being within an open temperature interval of the initial room temperature and the requested temperature setpoint, while adjusting the color temperature of the at least one light fixture (30) according to the requested temperature setpoint.

11. A method of any preceding claims, the method being controlled on premises of the at least one room (10) or at a remote location via cloud platform services.

12. A method to any preceding claims, the method further comprising, controlling the HVAC device (20) to adjust the room temperature of the at least one room (10) if an additional temperature setpoint request is received via the temperature setpoint determining device (24) within a fifth time frame after the initial temperature setpoint request was received via the temperature setpoint determining device (24), the fifth time frame being greater than 5 minutes and/or less than 15 minutes, while the limits of fifth time frame are user adjustable.

13. A light fixture color temperature adjustment device for improving the cognitive thermal comfort, the device comprising:
at least one lighting control device (44, 46) for controlling the color temperature of
at least one light fixture (30);
wherein a temperature setpoint is received via a temperature setpoint determining device (24); and
the color temperature of the at least one light fixture (30) is adjusted within a first time frame according to the temperature setpoint.

14. A device of claim 13, the device further comprising a temperature setpoint determining device (24) and/or the light fixture (30).

15. A device of claims 13-14, the device further comprising at least one or more elements of the following group:
an illumination sensor (32), a temperature sensor (22), an HVAC device (20), an interconnect device (40), an HVAC control device (42);
wherein the room temperature of the at least one room (10) is preferably adjusted by the at least one HVAC device (20) which is controlled by the at least one HVAC control device (42); and
wherein the interconnect device (40) preferably interconnects the HVAC control device (42) and the lighting control device (44, 46).

16. A device of any preceding claims 14-15, wherein the current and/or planned use state of at least one room (10) within which the device is applied is determined.

17. A device of any preceding claims 13-16, wherein the adjustment of the room temperature of the at least one room (10) via the at least one HVAC device (20) is limited to the limited temperature setpoint, the limited temperature setpoint being within an open temperature interval of the initial room temperature and the requested temperature setpoint, while the color temperature of the at least one light fixture (30) is adjusted according to the requested temperature setpoint.
